# EUROPEAN PATENT APPLICATION

(11) **EP 3 862 049 A1**
(43) Date of publication of application: **11.08.2021**
(21) Application number: 20155450.8
(22) Date of filing: 04.02.2020
(51) Int. Cl.: A61N 1/375, A61N 1/39

(54) **NON-TRANSVENOUS IMPLANTABLE CARDIOVERTER-DEFIBRILLATOR**

(71) Applicant: BIOTRONIK SE & Co. KG, 12359 Berlin (DE)
(72) Inventor: Dörr, Thomas, 12437 Berlin (DE); Weiss, Ingo, 12435 Berlin (DE); Lang, Volker, 12161 Berlin (DE)
(74) Representative: Biotronik Corporate Services SE

(57) **Abstract**

The invention relates to a non-transvenous implantable cardioverter-defibrillator (1), comprising a housing having a length (L), a width (B) and a depth (D), The cardioverter-defibrillator (1) is characterized in that a ratio of the width (B) to the length (L) is smaller than or equal to 0.75.

## Description

The present invention relates to a non-transvenous implantable cardioverter-defibrillator according to the preamble of claim 1 and to a cardioverter-defibrillator system comprising such a non-transvenous implantable cardioverter-defibrillator according to the preamble of claim 11.

Conventional cardioverter-defibrillators (ICDs) are typically implanted in an upper chest region and comprise an electrode, which is implanted through a vein leading to the heart, and in particular through the superior vena cava, into the right ventricle. Such ICDs are therefore also referred to as transvenous ICDs.

In addition, non-transvenous ICDs are known, which are also referred to as non-transvenous implantable cardioverter-defibrillators. For example, Boston Scientific offers a non-transvenous ICD system in the market under the designation Emblem S-ICD. This non-transvenous ICD comprises a cuboid housing having a length of 83.1 mm, a width of 69.1 mm, and a depth of 12.7 mm. This results in a width-to-length ratio of 0.83.

Non-transvenous ICDs are typically implanted between the latissimus dorsi muscle (large muscle on the back) and the serratus anterior muscle (front muscle having saw-like appearance). After implantation of the non-transvenous ICDs already available in the market, the housing contour typically projects slightly laterally from the surrounding tissue on the outer sides. These housing regions are subject to tissue tension to a greater extent. This may cause necroses or infections. Moreover, the housing shape of the non-transvenous ICDs available in the market brings with it the risk for the so-called Twiddler's syndrome due to insufficient fixation of the ICDs in the tissue of the affected patients.

So as to circumvent this problem, the non-transvenous ICDs available in the market are, at times, implanted in implantation sites that ensure more reliable implantation, but are less favorable with respect to the subsequent defibrillation action.

It is the object of the present invention to provide a non-transvenous ICD that is improved compared to the non-transvenous ICDs available in the market and that is more favorable to implant anatomically, while nonetheless exhibiting good defibrillation action.

This object is achieved by a non-transvenous implantable cardioverter-defibrillator (ICD) having the features of claim 1. Such an ICD comprises a housing having a length, a width and a depth. The length represents the largest extension of the housing. It extends along a first direction. The width represents the second largest extension of the housing. It extends in the same plane as the length along a second direction, wherein the first direction and the second direction extend perpendicularly to one another. The depth, which may also be referred to as thickness, finally represents the third largest extension of the housing and extends along a third direction in a plane extending perpendicularly to the plane of the length and width. If the extension of the housing of the non-transvenous ICD is now projected into a Cartesian coordinate system, the length extends along the x axis, the width extends along the y axis, and the depth extends along the z- axis.

According to the invention, the non-transvenous ICD is characterized in that a ratio of the width to the length (that is, the width divided by the length) is smaller than or equal to 0.75, in particular smaller than or equal to 0.7, in particular smaller than or equal to 0.6, in particular smaller than or equal to 0.5, and in particular smaller than or equal to 0.4 The ratio of the width to the length can, for example, be in a range of 0.3 to 0.75, in particular of 0.4 to 0.7, and in particular of 0.5 to 0.6.

Such a configuration of the housing allows the non-transvenous ICD to be implanted in an anatomically favorable manner between the latissimus dorsi muscle and the serratus anterior muscle, wherein an electrically effective surface of the housing can be implanted as far dorsally as possible. The non-transvenous ICD having the dimensions claimed according to the invention can be implanted with considerably less tissue stress than the non-transvenous ICDs available in the market, thereby resulting in improved embedding of the non-transvenous ICD. This significantly reduces or entirely avoids the risk with respect to Twiddler's syndrome.

As a result of the ratio of the width to the length of the housing of the non-transvenous ICD claimed according to the invention, a sufficiently large ICD can still be implemented. Even non-transvenous ICDs require a particular minimum volume since in particular the battery, which supplies the energy required for a certain number of defibrillation shocks, necessitates significant space.

In one variant, at least one region of the housing is provided and configured to serve as an electrode during operation of the non-transvenous ICD. In particular, it is provided that this region of the housing serves as the shock electrode, which emits a defibrillation shock. It is possible to design the entire housing as an electrode. It is also possible to design only the region of the housing which faces the interior of the body in the implanted state as an electrode. Furthermore, it is contemplated in one variant to design only a section of the region of the housing facing the interior of the body which is arranged dorsally in the implanted state of the non-transvenous ICD as an electrode, for example a section that extends over up to 75%, in particular up to 60%, in particular up to 50%, in particular up to 40%, and in particular up to 30% of the surface area of the non-transvenous ICD facing the interior of the body. For example, a region that accounts for 25% to 75%, in particular 30% to 70%, and in particular 40% to 60% of the surface area of the non-transvenous ICD facing the interior of the body can be designed as an electrode, while the remaining regions are not designed as an electrode.

In one embodiment, the non-transvenous ICD is produced in different variants having differing lengths. It is then possible, during implantation, to select a configuration of the non-transvenous ICD that is particularly suitable for the anatomical situation of the patient in whom the non-transvenous ICD is to be implanted. A length of the non-transvenous ICD is selected, in particular, based on the length of the left ventricle of the heart of the patient in whom the ICD is to be implanted. The reason is that it is favorable when the length of the non-transvenous ICD corresponds to the length of the left ventricle of the heart.

So as to ensure good success of the implantation, it is necessary not to design the non-transvenous ICD too wide. In one embodiment, the width of the housing is thus no more than 60 mm, in particular no more than 55 mm, in particular no more than 50 mm, and in particular no more than 45 mm. The width of the housing can, for example, be in a range of 35 to 60 mm, in particular of 40 mm to 55 mm, and in particular of 45 mm to 50 mm.

In one embodiment, the non-transvenous ICD comprises a header used to connect an electrode. Such an electrode is typically used as a counter electrode and is implanted subcutaneously in a central chest region. The heart of a patient is then located exactly between the housing of the non-transvenous ICD and the counter electrode, so that a defibrillation shock is able to act on the heart of the patient with a particularly advantageous vector when the patient requires such a defibrillation shock. The further dorsally the housing of the non-transvenous ICD is implanted, the more favorable is the vector of the electric field.

In one variant, the header is arranged on a narrow side of the housing. The narrow side extends along the width of the housing. In another variant, the header is arranged along a longitudinal side of the housing. The longitudinal side of the housing extends along the length of the housing. Both in the one and in the other variant, it is possible to adapt the header to the outer contour of the housing so that the header conforms to the outer contour of the housing and, essentially, does not protrude in relation to the outer housing contours. It is then possible for an implantation of the housing to take place that causes particularly little tissue damage. However, as an alternative, it is also possible in both variants that the header protrudes in relation to the outer contours of the housing. Such an arrangement of the header has the advantage that an electrode inserted into the header can then be guided particularly well along the outer contour of the housing, and thus be stabilized. This can result in an implantation of the housing, together with the electrode, that causes little tissue damage.

In another variant, it is provided that the header extends both along the longitudinal side and along the narrow side of the housing. It is also possible in this variant to either integrate the header within the outer contours of the housing or to have it protrude in relation to the outer contours of the housing.

The header is typically arranged on the housing so as not to protrude in relation to the remaining housing parts in the depth direction of the housing, but, for example, to adjoin the remaining housing parts flush.

The housing of the non-transvenous ICD typically does not have any sharp edges so as to minimize the risk of tissue damage after an implantation of the ICDs to as great an extent as possible. For example, the housing of the non-transvenous ICD can have a rectangular basic shape, so as to then have a cuboid configuration (having rounded edges). In one alternative, the housing has an elliptic base surface so as to have an overall cylindrical shape. An embodiment having a circular base surface, however, is not possible since the ratio of the width to the length of smaller than or equal to 0.75, as claimed according to the invention, can then not be achieved. By rounding the edges, the housing of the non-transvenous ICD can have the appearance of a piece of soap, resulting in an implantation option that causes particularly little tissue damage.

In one embodiment, the housing has a volume that is no more than 75 cm³, in particular no more than 60 cm³, in particular no more than 50 cm³, and in particular no more than 40 cm³. For example, the housing can have a volume in a range of 30 cm³ to 75 cm³, in particular 40 cm³ to 70 cm³, and in particular 50 cm³ to 60 cm³. Such a housing volume still ensures sufficient space for a battery inside the non-transvenous ICD. At the same time, tissue damage typically decreases as the size of the housing of the non-transvenous ICD decreases.

In one embodiment, the depth of the housing is no more than 15 mm, in particular no more than 14 mm, in particular no more than 13 mm, in particular no more than 12 mm, in particular no more than 11 mm, and in particular no more than 10 mm. For example, the depth of the housing can be in a range of 8 mm to 15 mm, in particular of 9 mm to 14 mm, in particular of 10 mm to 13 mm, and in particular of 11 mm to 12 mm.

Non-transvenous ICDs typically have to be able to deliver defibrillation shocks having a higher energy than transvenous ICDs since the efficiency of transvenous ICDs is higher as a result of the arrangement of the shock electrode thereof in the heart. In one embodiment, the non-transvenous ICD thus comprises a defibrillation unit capable of delivering a defibrillation shock having a maximum shock energy of more than 45 J, in particular more than 55 J, in particular more than 65 J, and in particular more than 70 J. For example, the maximum shock energy that the defibrillation unit is able to provide for a defibrillation shock is in a range of 45 J to 120 J, in particular of 55 J to 110 J, in particular of 65 J to 100 J, and in particular of 70 J to 90 J.

In one variant, a surface of the housing, which faces an exterior of the body of the patient in the implanted state of the non-transvenous ICD, has a convex shape. In this way, it is possible to better adapt to the outside body contour of a patient, thereby making the wearing comfort of the ICD thus designed particularly high in the implanted state.

In one embodiment, a surface of the housing, which faces an interior of the body of a patient in the implanted state of the non-transvenous ICD, has a concave shape. This embodiment may be combined with the above-described embodiment. This reduces the space requirement of the non-transvenous ICD toward the interior of the body even further so as to be less noticeable in the implanted state. Moreover, in this variant, the ICD can be implanted particularly favorably along the ribs of a patient, whereby it protrudes even less in the edge regions thereof than with a straight configuration of the side of the housing facing the interior of the body.

One aspect of the present invention relates to a cardioverter-defibrillator system comprising a non-transvenous implantable cardioverter-defibrillator according to the above description. This cardioverter-defibrillator is to be implanted subcutaneously or intramuscularly. This typically takes place on the left thoracic side, in particular between the latissimus dorsi muscle and the serratus anterior muscle. Moreover, the cardioverter-defibrillator system comprises an electrode to be implanted subcutaneously, which is electrically conductively connected to a defibrillation unit of the non-transvenous ICD. This connection is typically implemented by means of a header formed on the housing of the ICD. As was already mentioned above, the electrode is provided, in particular, for the subcutaneous implantation in a central chest region so that, after the implantation of the cardioverter-defibrillator system, the heart of the patient is located between the housing of the cardioverter-defibrillator system and the subcutaneously implanted electrode, which acts as the counter electrode.

All variants and embodiments of the described non-transvenous ICD can be arbitrarily combined with one another and applied individually or in any arbitrary combination to the described cardioverter-defibrillator system, and vice versa.

Further details of aspects of the present invention will be described in more detail based on exemplary embodiments and figures. In the drawings:
- FIG. 1A: shows a schematic side view of a thorax of a person from the left side;
- FIG. 1B: shows the thorax of FIG. 1A with the indicated implantation site of a non-transvenous ICD;
- FIG. 2A: shows an enlarged detailed illustration of the implantation region of the non-transvenous ICD of FIG. 1B;
- FIG. 2B: shows a schematic sectional view through a non-transvenous ICD in the implanted state along a rib of a patient;
- FIG. 3B: shows a first exemplary embodiment of an ICD housing comprising a header; and

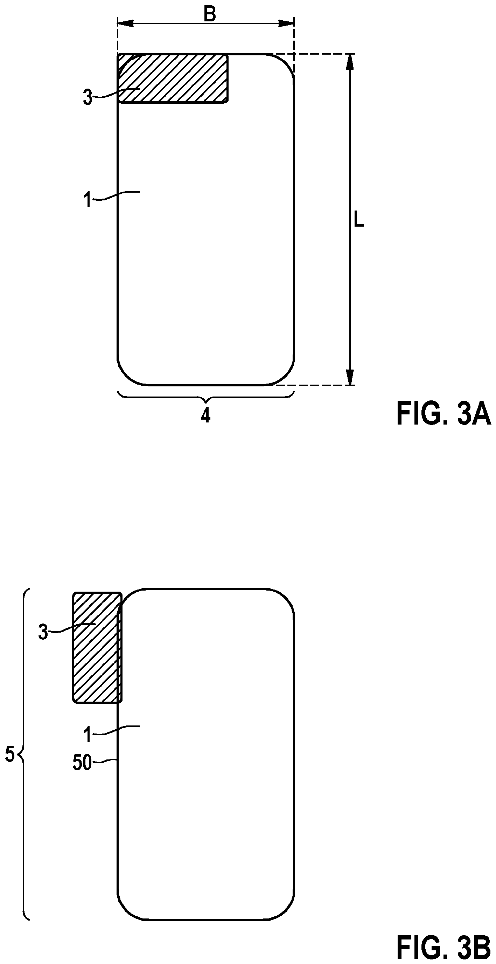

- FIG. 3B: shows a second exemplary embodiment of an ICD housing comprising a header.

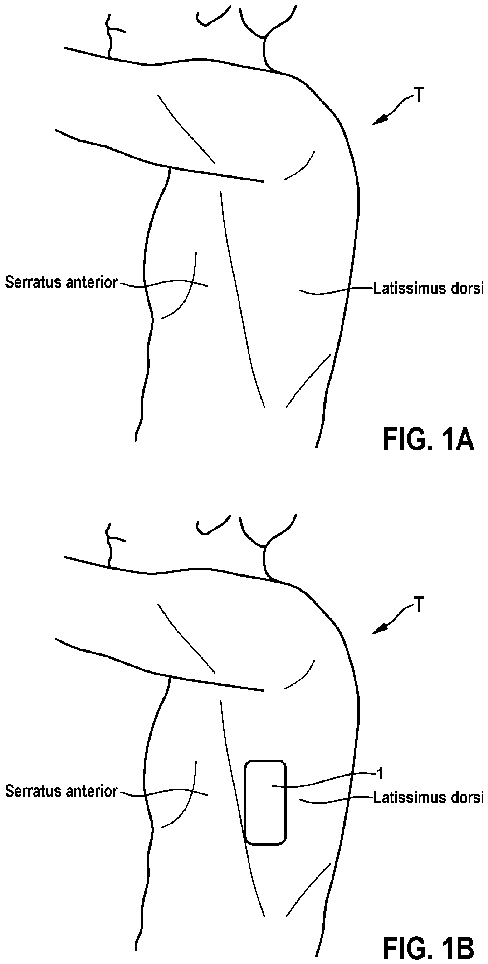

FIG. 1A shows a thorax T of a patient in a side view. The anatomical layers of the latissimus dorsi muscle and of the serratus anterior muscle are marked.

FIG. 1B shows a preferred implantation site for a non-transvenous cardioverter-defibrillator 1 (ICD) between the latissimus dorsi muscle and of the serratus anterior muscle. The non-transvenous ICD 1 is implanted subcutaneously or intramuscularly and shown only schematically in the representation of FIG. 1B, so as to be able to better visualize the implantation site thereof. Only a housing of the ICD 1 is shown.

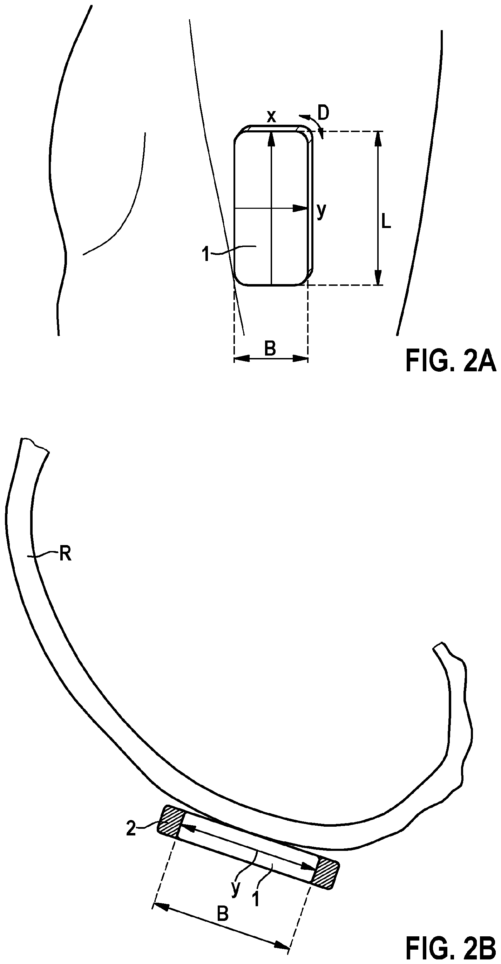

FIG. 2A shows an enlarged detailed illustration of the implantation site of the non-transvenous ICD 1 from FIG. IB. Comparable elements are always denoted by the same reference numerals in this figure and those that follow.

The housing of the non-transvenous ICD 1 has a length L along a longitudinal direction x, a width B along a width direction y extending perpendicularly to the longitudinal direction x, and a depth or thickness D extending along a plane spanned perpendicularly to the longitudinal direction x and the width direction y into the paper plane. The ratio of the width B to the length L is less than 0.75. In this way, it is possible for the non-transvenous ICD 1 to nestle better against an anatomical structure than is the case with non-transvenous ICDs known from the prior art. This is shown in detail in FIG. 2B.

Fig. 2B shows a rib R of a patient in a view from above. The illustration of FIG. 2B is cut in a plane extending above the rib R. The non-transvenous ICD 1 is implanted in a dorsal region of the rib R. For comparison purposes, the extensions of a non-transvenous ICD 2 known from the prior art are likewise shown. The non-transvenous ICD 1 has a width B extending along the width direction y, which is significantly reduced compared to the widths of the non-transvenous ICDs known from the prior art. As a result, the non-transvenous ICD 1 protrudes less in relation to the anatomical structure of the rib R of the patient, whereby it can become more easily embedded into the tissue of the patient, and the implantation thereof results in less tissue damage than the implantation of a non-transvenous ICD 2 known from the prior art.

FIG. 3A shows a variant of the non-transvenous ICD 1 in which a header 3, which is used to connect an electrode, is arranged on a narrow side 4 of the housing which extends along the width B of the non-transvenous ICD 1. The header 3 conforms to the outer contours of the housing of the non-transvenous ICD 1 in the process. As an alternative, it could also protrude in relation to the outer contours of the housing of the non-transvenous ICD 1.

Such an arrangement is represented by way of example in FIG. 3B. In this arrangement of the header 3 on a longitudinal side 5 of the housing of the non-transvenous ICD 1, the header 3 does not conform to the outer contour of the housing of the non-transvenous ICD 1, but protrudes over the outer contour of the housing. In this embodiment, it is contemplated that an electrode is inserted from beneath into the header 3, so that the electrode can be supported along the section 50 of the longitudinal side 5 of the housing of the non-transvenous ICD 1 which remains beneath the header 3.

Both in the illustration of FIG. 3A and in the illustration of FIG. 3B, the header 3 does not protrude further over the housing of the non-transvenous ICD 1, out of the paper plane or into the paper plane, but in each case ends flush with the corresponding housing sides of the non-transvenous ICD 1.

## Claims

1. A non-transvenous implantable cardioverter-defibrillator, comprising a housing having a length (L), a width (B) and a depth (D),
**characterized in that**
a ratio of the width (B) to the length (L) is smaller than or equal to 0.75.

2. The non-transvenous implantable cardioverter-defibrillator according to claim 1, **characterized in that** at least one section of the housing is provided and configured to serve as an electrode during operation of the cardioverter-defibrillator (1).

3. The non-transvenous implantable cardioverter-defibrillator according to claim 1 or 2, **characterized in that** the width (B) of the housing is no more than 60 mm.

4. The non-transvenous implantable cardioverter-defibrillator according to any one of the preceding claims, **characterized in that** the cardioverter-defibrillator (1) comprises a header (3) that is arranged on a narrow side (4) of the housing and used to connect an electrode.

5. The non-transvenous implantable cardioverter-defibrillator according to any one of the preceding claims, **characterized in that** the cardioverter-defibrillator (1) comprises a header (3) that is arranged on a longitudinal side (5) of the housing and used to connect an electrode.

6. The non-transvenous implantable cardioverter-defibrillator according to any one of the preceding claims, **characterized in that** the housing has a volume of no more than 75 cm³.

7. The non-transvenous implantable cardioverter-defibrillator according to any one of the preceding claims, **characterized in that** the depth (D) of the housing is no more than 15 mm.

8. The non-transvenous implantable cardioverter-defibrillator according to any one of the preceding claims, **characterized in that** the cardioverter-defibrillator (1) comprises a defibrillation unit that is capable of delivering a defibrillation shock having a maximum shock energy of more than 45 J.

9. The non-transvenous implantable cardioverter-defibrillator according to any one of the preceding claims, **characterized in that** a surface of the housing, which faces an exterior of the body of a patient in the implanted state of the cardioverter-defibrillator (1), has a convex shape.

10. The non-transvenous implantable cardioverter-defibrillator according to any one of the preceding claims, **characterized in that** a surface of the housing, which faces an interior of the body of a patient in the implanted state of the cardioverter-defibrillator (1), has a concave shape.

11. A cardioverter-defibrillator system comprising a non-transvenous implantable cardioverter-defibrillator (1) according to any one of the preceding claims, and a subcutaneously implantable electrode, which is electrically conductively connected to a defibrillation unit of the non-transvenous implantable cardioverter-defibrillator (1).
